Europäisches Patentamt

European Patent Office

Office européen des brevets

(19)

(11) Veröffentlichungsnummer: **0 056 602**
**A1**

(12) **EUROPÄISCHE PATENTANMELDUNG**

(21) Anmeldenummer: **82100155.9**

(22) Anmeldetag: **12.01.82**

(51) Int. Cl.³: **A 61 F 1/04**

(30) Priorität: **15.01.81 DE 3101087**

(43) Veröffentlichungstag·der Anmeldung:
**28.07.82 Patentblatt 82/30**

(84) Benannte Vertragsstaaten:
**AT DE FR GB IT NL SE**

(71) Anmelder: **Krieg, Bernd**
**Flüggestrasse 16**
**D-2000 Hamburg 60(DE)**

(72) Erfinder: **Krieg, Bernd**
**Flüggestrasse 16**
**D-2000 Hamburg 60(DE)**

(54) **Künstliches Kniegelenk mit hydraulischer Pendel- und Standphasensteuerung.**

(57) Das künstliche Kniegelenk enthält einen hinter der Kniegelenkachse (4) angelenkten Hydraulikzylinder (7), der als einfacher Schubkolbentrieb mit einem Kolben (19), einer Kolbenstange (18) und einem Arbeitszylinder (20) versehen ist und als wesentliches Bauelement ein frei drehbar aufgehängtes Pendel (22) oder eine auf einer geraden Bahn frei bewegliche Kugel enthält. Pendel (22) oder Kugel sind jeweils in der Gehebene im Hydraulikzylinder angeordnet und ihre rückwärtige Bewegung wird zum Unterbrechen des Hydraulikölstroms genutzt.

Die Kugel ist auf einer geraden Bahn im Hydraulikzylinder gelagert, die annähernd waagerecht zum künstlichen Bein liegt. Die Bahn der Kugel enthält auf Höhe des Kugelmittelpunktes und entgegen der Gehrichtung eine Austrittsbohrung für das Hydrauliköl von der Beugedruckseite des Hydraulikzylinders.

Das Pendel (22) wird beispielsweise in der Kolbenstange (18) des Hydraulikzylinders drehbar mittels einer Achse (42) in einer verschiebbaren Pendelhülse (21) aufgehängt und verschließt in Schrittvorlageposition des Beines mit seiner ebenen Dichtfläche (22b) ein Röhrchen (43), das Verbindung von der Beugedruckseite (35) des Hydraulikzylinders zum Arbeitszylinderraum (45) oberhalb des Kolbens hat.

Gehrichtung

Fig. 2

Bernd Krieg
Flüggestraße 16, 2000 Hamburg 60

0056602

## Künstliches Kniegelenk mit hydraulischer Pendel- und Standphasensteuerung

Die Erfindung bezieht sich auf ein künstliches Kniegelenk mit hydraulischer Pendel- und Standphasensteuerung. Zu einer vollständigen Prothese mit Oberschenkelschaft oder Hüftgelenk mit Hüftkorb und Unterschenkel mit Fuß ergänzt ermöglicht es einem am Oberschenkel sowie im Hüft- oder Kniegelenk amputierten Menschen ein sicheres und bei günstigen Stumpfverhältnissen weitgehend physiologisch erscheinendes Gehen. Während des Gangzyklus des Kunstbeins dämpft ein Hydraulikzylinder die Unterschenkeldrehung relativ zum Oberschenkel beim Durchschwingen und sichert die Standphase, in der das künstliche Kniegelenk die Körperlast trägt.

Es sind Beinprothesen bekannt, die im künstlichen Kniegelenk einen Hydraulikzylinder enthalten, der sowohl die Unterschenkeldrehung in der Pendelphase dämpft als auch das künstliche Kniegelenk in der Standphase gegen Einknicken sichert. Für die hydraulische Standphasensicherung haben sich zwei Steuerprinzipien praktisch bewährt:

1. Ein vom künstlichen Fuß ausgehendes Steuergestänge schließt bei Fersenbelastung ein Absperrorgan des Hydraulikzylinders, so daß dieser die Kniegelenkbewegung blockiert. Ein Nachteil dieser Lösung besteht jedoch darin, daß für die hydraulische Standphasensteuerung ein spezielles Fußgelenk und ein mechanisch relativ aufwendiges Steuergestänge erforderlich ist. Der Kunstfußtyp kann nicht frei gewählt werden und Kunstfüße mit bisher bewährten Fußgelenken sind nicht einsetzbar.

2. Beim zweiten Steuerprinzip wird ein Absperrorgan des Hydraulikzylinders ohne ein vom Fuß ausgehendes Steuergestänge durch das beim Gehen im künstlichen Kniegelenk auftretende Streck- und Beugemoment und dessen gangzyklusabhängigen zeitlichen Verlauf gesteuert. Für diese Standphasensteuerung ist neben der Pendelphasendämpfung ein zweiter Kolben mit einem Zylinder erforderlich. Weitere wesentliche Bauelemente der Standphasensteuerung sind ein Rückschlagventil mit einem Ventilschaft, ein mit einer Unwucht versehenes Pendelrädchen mit einem Segment zum Blockieren des Ventilschaftes, ein Gegengewicht zum

Anheben des Pendelrädchens, eine Steuerkante zum Anheben des Gegengewichts und eine Tellerfeder zum Vermeiden einer längerdauernden Überstreckung des Kniegelenks am Ende der Pendelphase der Prothese. Das funktionsgerechte Zusammenwirken dieser Bauelemente hängt von der richtigen Abstimmung der wirksamen Drehmomente von Pendelrädchen und Gegengewicht, der Rotationszeit des Pendelrädchens, dem Reibschluß zwischen Rückschlagventilschaft und Pendelsegment und dem von der Hydraulikflüssigkeit auf das Rückschlagventil wirkenden Strömungsdruck ab. Ein Nachteil dieser Lösung besteht darin, daß die Anordnung der Steuerelemente für die Standphasensteuerung einen besonderen Kolben mit Zylinder erfordert und zur Betätigung des Absperrorgans mehrere präzise aufeinander abgestimmte Funktionselemente zusammenwirken müssen. Die hydraulische Standphasensteuerung erfordert zur Herstellung einen dementsprechend großen Fertigungsaufwand und verlängert den Hydraulikzylinder um das Maß des Standphasenzylinders. Dies hat zur Folge, daß die relativ große Bauhöhe das Anbringen eines Winkeljustieradapters unterhalb des Kniegelenks bei Rohrskelettprothesen behindert und der gesamte Hydraulikzylinder eine relativ große Masse aufweist. Das Ziel einer guten Prothesenversorgung, eine möglichst leichte Prothese zu bauen, die optimal an die individuellen Gegebenheiten des Versehrten angepaßt werden kann, läßt sich bedingt durch Masse und Bauhöhe des Hydraulikzylinders nicht bei jeder Prothesenversorgung realisieren.

Aufgabe der Erfindung ist es, die dargestellten Nachteile zu vermeiden und Bauabmessungen, Masse und mechanischen Aufwand für die hydraulische Pendel- und Standphasensteuerung auf ein geringstmögliches Maß zu reduzieren bei üblichem Hub und üblichem Arbeitszylinderinnendurchmesser des Hydraulikzylinders. Die hydraulische Pendel- und Standphasensteuerung soll auf einen einfachen Schubkolbentrieb reduziert werden, der ein kombiniertes Pendel- und Standphasensteuerventil als eine Einheit in sich vereinigt und kein vom Fuß ausgehendes Steuergestänge zur Standphasensicherung benötigt.

Die Lösung der gestellten Aufgabe beruht auf folgender Idee: Ein Hydraulikzylinder als Schubkolbentrieb ist in seiner Lage zum Bein während der gesamten Belastungsphase der Prothese als ortsfest in bezug auf diese anzusehen. In bezug auf einen außenstehenden Beobachter macht der Hydraulikzylinder die

0056602

gleiche Neigungsbewegung während der Belastungsphase des Kunstbeins wie dieses. Ein drehbar aufgehängtes Pendel oder eine auf einer geraden Bahn frei bewegliche Kugel, die jeweils nur der Schwerkraft gehorchen, können im Hydraulikzylinder angeordnet werden und infolge der Beinneigung nach vorne in der Schrittvorlage durch ihre unabhängige Ausrichtung nach der Schwerkraft eine Ventilöffnung schließen und diese Öffnung infolge der Beinneigung nach hinten in der Schrittrücklage wieder freigeben.

Da die Stellgenauigkeit eines frei drehbaren und relativ langen Pendels naturgemäß wesentlich größer ist als die einer frei beweglichen Kugel, die sich auf einer geraden Bahn befindet, wird im ersten Ausführungsbeispiel der Standphasensteuerung durch ein frei drehbares Pendel der Vorzug gegeben.

Konstruktiv wird die Aufgabe der Erfindung dadurch gelöst, daß ein Pendel frei drehbar im oberen Ende einer oben geschlossenen zylindrischen Hülse gelagert ist, daß das Pendel mit einer am unteren Ende in der Gehebene nach rückwärts angeordneten ebenen Dichtfläche versehen ist, daß die Hülse dieser Dichtfläche gegenüberliegend eine Austrittsbohrung für das Hydrauliköl mit einem kurzen Röhrchen und einer kreisringförmigen Dichtfläche enthält, daß die nach unten offene Hülse mit ihrer unteren kreisringförmigen Stirnfläche eine Eintrittsbohrung von der Beugedruckseite des Hydraulikzylinders freigibt und daß die Hülse in Achsrichtung verschiebbar, aber nicht drehbar gelagert ist und daß durch Verschieben der Hülse in Achsrichtung mittels einer Stellschraube je nach Konstruktion des Hydraulikzylinders entweder die Eintritts- oder Austrittsbohrung teilweise abgedeckt wird und somit eine spaltblendenähnliche Drosselung zur Dämpfung der Pendelphasenbewegung des Unterschenkels entsteht.

Eine andere konstruktive Lösung des Erfindungsgedankens besteht darin, daß in einem Hydraulikzylinder eine Kugel auf einer in der Gehebene liegenden und in Stand- oder leichter Schrittrücklageposition des künstlichen Beins zu diesem waagerecht angeordneten geraden Bahn frei beweglich gelagert ist, daß die gerade Bahn der Kugel hinten (in der Gehebene) durch eine Austrittsbohrung begrenzt wird, die auf Höhe des Kugelmittelpunktes der geraden Bahn angeordnet ist und daß der Weg des Hydrauliköls von der Beugedruckseite des Hydraulikzylinders vom Raum der Kugel aus durch diese Austrittsbohrung führt, so daß die Kugel infolge der Beinneigung in der Schrittvorlage vor die Austrittsbohrung rollt, diese verschließt und somit die Beugebewegung des Kniegelenks blockiert und infolge der entgegengesetzten Beinneigung in der Schrittrücklage wieder freigibt. Weiterhin sieht diese konstruktive Lösung eine Drosselschraube in Achsrichtung der Austrittsbohrung vor, die den freien Querschnitt der Austrittsbohrung verändern kann, so daß die Beugebewegung des Hydraulikzylinders in der Pendelphase gedämpft wird.

Baugröße und Masse des Hydraulikzylinders werden dadurch auf ein Minimum begrenzt, daß für die hydraulische Dämpfung der Streck- und Beugebewegung bei der Standphasensteuerung mittels Pendelventil oder Kugel das Schaltungsprinzip der einseitig wirkenden oder doppeltwirkenden Kolbenpumpe verwendet wird, wobei der notwendige Umström- und Kompensationsraum jeweils durch ein den Arbeitszylinder umschließendes Ringvolumen gebildet wird.

Die Erfindung wird im einzelnen anhand der Zeichnungen erläutert, in denen ein Ausführungsbeispiel und zwei Lösungswege des Erfindungsgedankens dargestellt sind. Es zeigt

Fig.1 das künstliche Kniegelenk mit Hydraulikzylinder in der Seitenansicht in natürlichen Abmessungen,

Fig.2 eine vergrößerte Schnittdarstellung des Hydraulikzylinders, dargestellt in der Gehebene, wobei hier die konstruktive Lösung mittels Pendelventil dargestellt wird,

Fig.3 eine Schnittdarstellung der Kolbenstangenanlenkung des Hydraulikzylinders in der Frontalebene,

Fig.4 einen Teilschnitt des Hydraulikzylinders in der Horizontalebene.

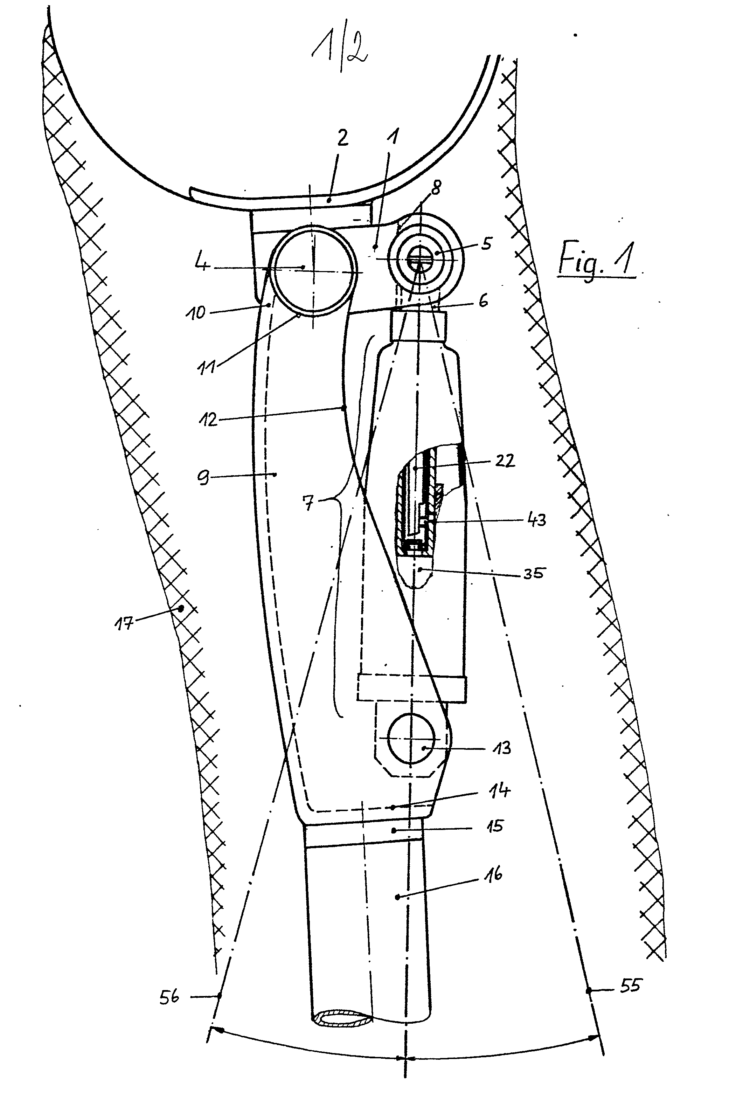

Das Gelenkoberteil 1 des künstlichen Kniegelenks (Fig.1) ist durch einen Schaftansatz 2 mit dem Oberschenkelschaft 3 des

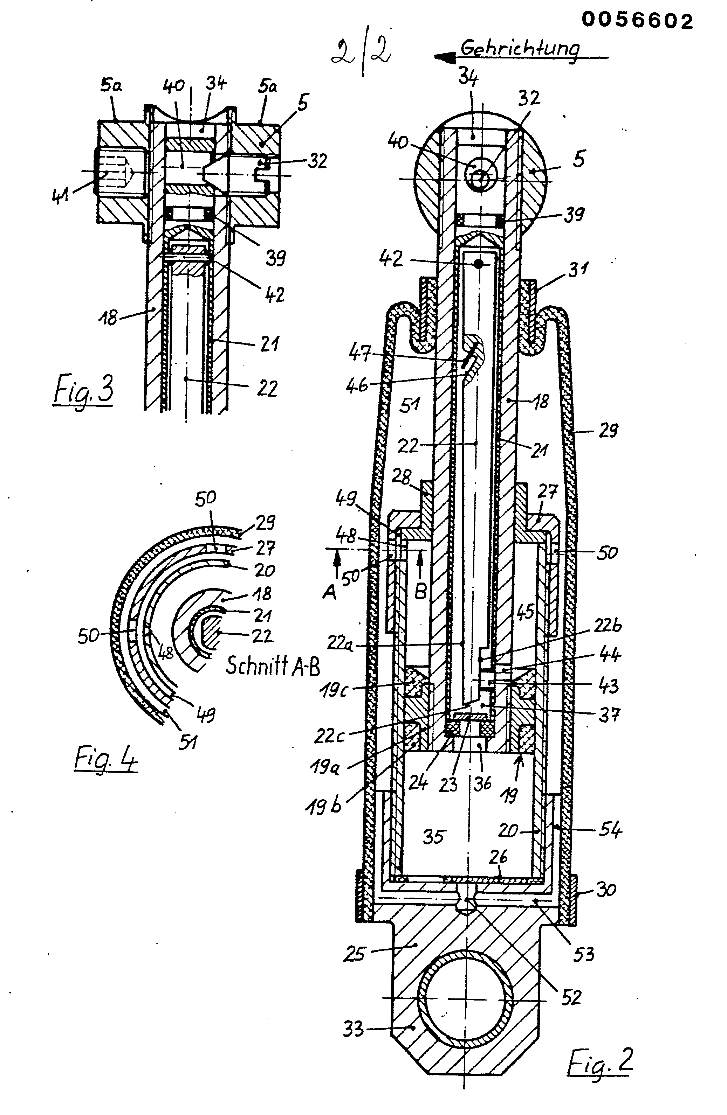

Kunstbeins lösbar verbunden. Das Gelenkoberteil 1 enthält die Knieachse 4 und hinter der Knieachse angeordnet einen oberen Achsbolzen 5 zur Anlenkung der Kolbenstange 6 des Hydraulikzylinders 7 (Fig. 1). Der Achsbolzen 5 ist drehbar im Gelenkoberteil 1 gelagert. Die Kolbenstange 6 wird mittels eines Gewindes in den Achsbolzen 5 eingeschraubt (Fig. 2 und 3). Damit das obere Ende der Kolbenstange 6 beim Beugen des Kniegelenks im Gelenkoberteil freigeht, ist dieses von hinten mit einem mittig angeordneten Schlitz 8 versehen (Fig. 1). Die Knieachse 4 verbindet das Gelenkoberteil 1 drehbar mit einem Gelenkunterteil 9 (Fig. 1). Dieses besteht aus zwei Gabelenden 1o mit Lageraugen 11 zur Lagerung der Knieachse 4. Die lichte Breite zwischen den Gabelenden 1o ist so bemessen, daß der Hydraulikzylinder 7 in das Gelenkunterteil beim Beugen des Kniegelenks einschwenken kann. Um auch bei Knieexartikulationen einen ausreichenden Beugewinkel des künstlichen Kniegelenks zu gewährleisten, sind die oberen Enden der Gabel von hinten in der Gehebene betrachtet mit konkaven Rundungen 12 versehen (Fig. 1). Die Gabelenden 1o sind im oberen Bereich derartig schmal, daß hierdurch die Bauhöhe des Gelenkoberteils bei gebeugtem Kniegelenk nicht vergrößert wird. Das untere Ende des Gelenkunterteils enthält im hinteren Bereich einen unteren Achsbolzen 13 für die untere Anlenkung des Hydraulikzylinders 7 (Fig. 1). Der untere Achsbolzen 13 liegt lotrecht unter dem Achsbolzen 5 des Hydraulikzylinders. Das Gelenkunterteil 9 ist mit einer unteren Abschlußfläche 14 beispielsweise mittels eines Justieradapters 15 mit dem Unterschenkelrohr 16 verbunden. Die kosmetische Form des Kunstbeins wird in bekannter Weise durch eine kosmetische Schaumstoffverkleidung 17 erzeugt. Ober- und Unterteil des künstlichen Kniegelenks können auch ein sogenanntes "Holzpaßteil" zur Anfertigung von Holzprothesen in Schalenbauweise sein.

Der Hydraulikzylinder 7 (Fig. 1) besteht aus einer Kolbenstange 18, einem Kolben 19, einem Arbeitszylinder 2o, einer Pendelhülse 21 mit einem Pendel 22, einem Rückschlagventilplättchen 23 mit einer elastischen Scheibe 24, einem unteren Deckel 25 des Arbeitszylinders mit einer elastischen Rückschlagklappe 26, einem oberen Deckel 27 mit einer Führungs- und Dichtungsbuchse 28, einem Außenbalg 29 mit einer unteren Schelle 3o und einer oberen Schelle 31, einem oberen Achsbolzen 5 mit einer Stellschraube 32 und einem Lagerauge 33 für den unteren Achsbolzen 13. Die Kolbenstange 18 enthält von oben ausgehend eine Bohrung 34 und auf Höhe des unteren Bereichs vom Kolben 19 mit kleinerem Durchmesser abgestuft eine bis zum Arbeitszylinderraum 35 unterhalb des Kolbens 19 durchgehende Bohrung 36. Die Kolbenstange enthält in dem am unteren Ende befindlichen Absatz der Bohrung 34 eine elastische Scheibe 24 mit zentraler Durchgangsbohrung , die beispielsweise aus einem hochelastischen Polyurethan hergestellt ist. Auf dieser elastischen Scheibe aufliegend ist von oben in die Bohrung 34 die zylindrische Pendelhülse 21 eingeschoben. Diese enthält eine im oberen Ende endende Bohrung 37, so daß die untere Stirnfläche kreisringförmig ist. Auf der elastischen Scheibe 24 liegt in der Bohrung 37 der Pendelhülse das runde Rückschlagventilplättchen 23. Dies hat einen geringeren Durchmesser als die Bohrung 37, aber einen größeren Durchmesser als die Durchgangsbohrung der elastischen Scheibe 24, so daß diese beim Ausfahren des Kolbens vom Rückschlagventilplättchen abgedeckt wird und beim Einfahren des Kolbens zwischen Bohrung 37 und Außendurchmesser des Rückschlagventilplättchens eine ausreichende Ringfläche zum Durchströmen der Hydraulikflüssigkeit besteht.

Das obere Ende der Pendelhülse enthält oberhalb der Bohrung 37 eine Rille mit einem Dichtungsring 39 und darüber eine durchgehende Querbohrung 4o , die in Achsrichtung des oberen Achsbolzens 5 verläuft (Fig.1 und 2). Der Achsbolzen 5 enthält zwei zylindrische Lagerzapfen 5a mit verkleinertem Durchmesser (Fig.3). Die Lagerzapfen 5a sind axial mit Gewindebohrungen zur Aufnahme einer Arretierschraube 4o für die Kolbenstange und einer Stellschraube 32 für die Pendelhülse 21 versehen. Die Stellschraube 32 ist ein Gewindestift mit Kegelansatzspitze und greift mit diesem Ansatz in die Querbohrung 4o. Die Mittelachse der Querbohrung 4o ist um ein geringes Maß gegenüber der Mittelachse der Stellschraube 32 höher angeordnet, so daß die Kegelansatzspitze der Stellschraube durch Hereindrehen die Pendelhülse 21 am Rand der Querbohrung 4o angreifend nach unten drückt.

Die Pendelhülse 21 nimmt in ihrer Bohrung 37 das Pendel 22 auf. Dieses ist aus einem zylindrischen Querschnitt geformt, der Spiel in der Bohrung 37 hat. Das obere Ende des Pendels enthält eine drehbare Lagerung mittels einer zylindrischen Achse 42. Die Enden der Achse 42 sind mit der Pendelhülse 21 fest verbunden. Die Achse 42 ist waagerecht und quer zur Gehebene angeordnet, so daß das Pendel in der Gehebene drehbar ist. Die Vorderseite 22a des Pendels enthält eine sich nach unten verbreiternde keilförmige Abrundung, um einen ausreichenden Pendelausschlag in der Schrittrücklage des Kunstbeins zu gewährleisten. Am unteren Ende der Rückseite enthält das Pendel eine senkrecht verlaufende ebene Dichtfläche 22b. Die Pendelhülse 21 ist auf Höhe der Dichtfläche 22b des Pendels mit einem kurzen Röhrchen 43 versehen, dessen ringförmige Stirnfläche von der ebenen Dichtfläche 22b des Pendels 22 verschlossen werden kann.

Um ungefähr einen halben Bohrungsdurchmesser höhenversetzt zum Röhrchen 43 befindet sich in der Kolbenstange in der gleichen Ebene eine Durchgangsbohrung 44 (Fig.2). Diese Durchgangsbohrung hat Verbindung zum Arbeitszylinderraum 45 oberhalb des Kolbens 19. Wird die Stellschraube 32 mit ihrer Kegelansatzspitze weiter in die Querbohrung 4o der Pendelhülse 21 hineingedreht, so schiebt die Stellschraube die Pendelhülse 21 in der Kolbenstange 18 nach unten und der freie Querschnitt zwischen dem Röhrchen 43 und der Durchgangsbohrung 44 wird verkleinert. Dabei wird die elastische Scheibe 24 etwas zusammengedrückt. Dies hat keinen Einfluß auf die Dichtwirkung des Rückschlagventilplättchens 23, da die Verformung sich symmetrisch vollzieht und der Verstellhub sehr gering ist. Die Verkleinerung des freien Querschnitts zur Durchgangsbohrung 44 ruft eine spaltblendenähnliche Drosselwirkung hervor, so daß das Einfahren der Kolbenstange und somit die Beugebewegung des Kniegelenks in der Pendelphase (vgl. Fig.1) wirkungsvoll gedämpft werden kann. Eine Verstellbarkeit des freien Drosselquerschnitts dient dazu, die Dämpfungswirkung des Hydraulikzylinders optimal auf das Unterschenkelgewicht der Prothese und die Gehdynamik des Prothesenträgers abzustimmen.

Die untere Stirnfläche des Pendels 22 ist in der Weise schräggestellt, daß beim Durchströmen der Hydraulikflüssigkeit der Strömungsdruck in Zusammenwirken mit dem Rückschlagventilplättchen eine kleine Kraftkomponente in Öffnungsrichtung auf das Pendel ausübt (Fig.2). Auf diese Weise wird ein unerwünschtes Schließen des Pendels infolge Unterdruck beim Einfahren der Kolbenstange verhindert. An der Vorderseite (in Gehrichtung) ist das Pendel 22 im oberen Bereich unterhalb der Achse 42 mit einer rechtwinkligen Kerbe 46 und einem dünnen Federstahldrahtstift 47 versehen (Fig.2). Die obere schräge Fläche der Kerbe enthält eine Bohrung, in die das Ende des Federstahldrahtstiftes 47 eingeklebt ist. Der Federstahldrahtstift 47 zeigt mit seinem freien Ende schräg nach unten in Gehrichtung. Dreht das Pendel beim Neigen des künstlichen Beins in der Schrittrücklage in Gehrichtung, so begrenzt das Ende des Federstahldrahtstiftes 47 den Winkelweg des Pendels, indem das Stiftende an die Innenwandung der Pendelhülse anstößt. Aufgabe des Federstahldrahtstiftes 47 ist es, für ein rechtzeitiges Schließen des Röhrchens 43 durch die ebene Dichtfläche 22b des Pendels zu sorgen. Am Ende der Unterschenkeldrehung beim Durchschwingen des künstlichen Beins wird der Federstahldrahtstift 47 infolge der Massenträgheit des Pendels 22 elastisch verformt und sorgt mit Hilfe dieser gespeicherten Verformungsenergie für ein schnelleres Schließen des Röhrchens 43 durch die ebene Dichtfläche 22b des Pendels. Auf diese Weise ist das Kniegelenk beim Aufsetzen der Absatzkante des künstlichen Fußes gegen Einknicken gesichert.

Der Kolben 19 (Fig.2) besteht aus einer Gewindebuchse aus Metall 19a mit einem mittleren Ring mit Arbeitszylinderinnendurchmesser und zwei auf die im Durchmesser reduzierten Ansatzenden aufvulkanisierten Dichtringen 19b und 19c aus einem verschleißfesten Elastomer. Der Kolben 19 ist mit dem Innengewinde seiner Gewindebuchse 19a auf ein Gewindeende der Kolbenstange 18 aufgeschraubt. Der obere Dichtring 19c ist an seiner Stirnfläche zur Gewindebohrung hin konisch verjüngt. Diese Gestaltung hat zur Folge, daß der freie Querschnitt der Durchgangsbohrung 44 in der Kolbenstange 18 nicht eingeschränkt wird und bei Ballendrehung des Kunstfußes, wie es beim Abrollen in der Schrittrücklage der Fall ist, der obere Dichtungsring durch Anpressen an die untere Stirnfläche der Führungs- und Dichtungsbuchse 28 elastisch vorgespannt wird und beim Einbeugen des künstlichen Kniegelenks seine gespeicherte Verformungsenergie wieder abgibt. Dieser Vorgang unterstützt die Muskelarbeit des Versehrten beim Einbeugen des Kniegelenks in der Schrittrücklage.

Der Arbeitszylinder 2o ist mit Außengewinde versehen (Fig.2). Auf die Gewindeenden ist von oben der obere Deckel 27 mit der Führungs- und Dichtungsbuchse 28 und von unten der untere Deckel 25 mit dem Lagerauge 33 aufgeschraubt (Fig.2). Das obere Ende des Arbeitszylinders 2o enthält einen spaltförmigen Schlitz 48 (Fig.2 und 4) und einen am Außenumfang befindlichen Ansatz, der gegenüber dem Außendurchmesser verkleinert ist. Der Querschnitt des spaltförmigen Schlitzes 48 ist nach außen keilförmig erweitert, um eine spaltblendenähnliche Drosselwirkung zu erzielen. Am Ende des Arbeitszylinders entsteht am Außenumfang durch den Ansatz mit verkleinertem Durchmesser zwischen diesem und dem aufgeschraubten Deckel 27 ein Ringvolumen 49 (Fig.2 und 4). Dieses Ringvolumen ist durch Bohrungen 5o im Deckel 27 mit einem Kompensationsraum 51 verbunden. Bewegt sich der Kolben nach oben, so streckt sich das Kniegelenk und der obere Dichtungsring 19c des Kolbens deckt zunehmend den spaltförmigen Schlitz 48 ab. Da beim Ausfahren der Kolbenstange 18 das Rückschlagventilplättchen 23 (Fig.2) ein Ausweichen der Hydraulikflüssigkeit in den Arbeitszylinderraum 35 unterhalb des Kolbens verhindert, kann die Hydraulikflüssigkeit nur durch den spaltförmigen Schlitz 48 entweichen. Wird dieser zunehmend abgedeckt, so entsteht im Arbeitszylinderraum 45 oberhalb des Kolbens ein progressiv ansteigender Druck. Auf diese Weise kann die Streckbewegung des Unterschenkels am Ende der Pendelphase weich gedämpft werden, so daß ein stoßweises Kanten der gesamten Prothese infolge ruckartig abgefangener Massenkräfte vermieden wird.

Zwischen dem Deckel 25 und der unteren Stirnfläche des Arbeitszylinders 2o ist die elastische Rückschlagklappe 26 befestigt. Diese verschließt eine senkrechte Bohrung 52 mit zwei waagerechten seitlichen Bohrungen 53 und zwei senkrechten Kanälen 54 des Deckels 25 (Fig.2). Die Bohrungen 52 und 53 stehen über die senkrechten Kanäle 54 mit dem Kompensationsraum 51 in Verbindung. Bewegt sich der Kolben nach unten, so wird die elastische Rückschlagklappe 26 durch den Flüssigkeitsdruck im Arbeitszylinderraum 35 unterhalb des Kolbens 19 zugedrückt. Bewegt sich der Kolben nach oben, so vergrößert sich der Arbeitszylinderraum 35. Da das Rückschlagventilplättchen 23 das Überströmen von Hydrauliköl vom Arbeitzylinderraum 45 oberhalb des Kolbens 19 verhindert, entsteht im Arbeitszylinderraum 35 ein Unterdruck und es strömt Hydraulikflüssigkeit vom Kompensationsraum 51 durch die senkrechten Kanäle 54, die waagerechten Bohrungen 53 und die senkrechte Bohrung 52, hebt die elastische Rückschlagklappe 26 und ergänzt das Flüssigkeitsvolumen im Arbeitszylinderraum 35 unterhalb des Kolbens.

Der gesamte Hydraulikzylinder wird von dem elastischen Balg 29 umschlossen. Dieser hat einen kreisringförmigen Querschnitt, ist im Bereich des Arbeitszylinders zylindrisch, im mittleren Bereich der Kolbenstange leicht kegelstumpfförmig geformt und wird aus einem dauerfesten Elastomer hergestellt. Mit seinem unteren Ende ist der elastische Balg über den zylindrisch geformten Außenumfang des unteren Deckels 25 beschoben und wird

mit einer Schelle 3o gegen diesen Außenumfang gepreßt, so daß eine Abdichtung nach außen besteht. Das obere Ende des elastischen Balgs ist zylindrisch geformt und auf den Außendurchmesser der Kolbenstange 18 reduziert (Fig.1 und 2). Es wird mittels der Schelle 31 gegen die Kolbenstange gepreßt, so daß auch hier eine Abdichtung nach außen besteht. Fährt die Kolbenstange 18 in den Arbeitszylinder 2o ein, so verkürzt sich der elastische Balg 29 im Bereich der Kolbenstange, indem das an der Kolbenstange befestigte zylindrische Ende in den kegelstumpfförmigen Teil des elastischen Balgs eingezogen wird. Das Ringvolumen zwischen dem elastischen Balg 29 und dem Arbeitszylinder 2o bzw. der Kolbenstange 18 ist so hoch mit Hydraulikflüssigkeit angefüllt, daß ein im oberen Bereich befindliches Luftvolumen durch den Arbeitshub des Hydraulikzylinders beim Durchschwingen des Prothesenunterschenkels stark komprimiert wird und eine Vorbringerwirkung auf den Unterschenkel beim Strecken des Kniegelenks ausübt. Wird das künstliche Kniegelenk zum Hinsetzen gebeugt, so wird der gesamte elastische Balg 29 von dem durch die einfahrende Kolbenstange verdrängten Flüssigkeitsvolumen elastisch gedehnt. Aufgabe des elastischen Balgs ist somit zum einen die Kompensation der durch die Kolbenstange verdrängten Hydraulikflüssigkeit und zum anderen die Speicherung eines Teils der Beugeenergie zur Streckbeschleunigung des Prothesenunterschenkels in der Pendelphase. Eine weitere wichtige Aufgabe des elastischen Balgs besteht darin, den Hydraulikzylinder nach außen hermetisch abzudichten, so daß kein Flüssigkeitsverlust auftreten kann.

Die Wirkungsweise der hydraulischen Standphasensteuerung wird nun für die Belastungsphase der Prothese beschrieben. Berührt die Absatzkante des künstlichen Fußes den Boden, so ist das künstliche Bein schräg nach vorne geneigt und ein gedachtes Lot 55 von der Achse 42 des Pendels aus gefällt fällt hinter das Pendel 22. Die Gewichtskraft des Pendels erzeugt im Massenschwerpunkt angreifend ein linksdrehendes Drehmoment um die Pendelachse 42 und die ebene Dichtfläche 22b des Pendels verschließt das Röhrchen 43. Der Kolben 19 kann nicht einfahren, da das Hydrauliköl aus dem Arbeitszylinderraum 35 unterhalb des Kolbens nicht durch das Röhrchen 43 in den Arbeitszylinderraum 45 oberhalb des Kolbens entweichen kann. Auf diese Weise ist das Kniegelenk gegen Einbeugen gesichert und das durch die Fersenbelastung in dieser Belastungsphase der Prothese ausgelöste kniebeugende Drehmoment wird durch den Hydraulikzylinder
abgefangen. Nähert sich die belastete Prothese der Schrittrücklageposition, so ist sie schräg nach hinten geneigt und ein gedachtes Lot 56 von Achsmitte der Pendelachse 42 aus gefällt
fällt vor das Pendel. Die Gewichtskraft des Pendels erzeugt im
Massenschwerpunkt angreifend ein rechtsdrehendes Drehmoment um
die Pendelachse 42 und die ebene Dichtfläche 22b des Pendels
hebt vom Röhrchen 43 ab, sobald der Ballendruck des Kunstfußes
ein kniestreckendes Drehmoment erzeugt und den Arbeitszylinderraum 35 unterhalb des Kolbens 19 vom Beugedruck entlastet, so
daß kein Schließdruck mehr auf die Dichtflächenprojektion der
ebenen Dichtfläche 22b des Pendels wirkt. Am Ende der Standphase kann das Kniegelenk willkürlich gebeugt werden. Da sich
die Neigung des Hydraulikzylinders nach hinten beim Beugen des
Kniegelenks noch vergrößert, besteht keine Gefahr einer plötzlichen Blockierung der Beugebewegung durch Schließen des Pendels.

Die durch die Erfindung erreichten Vorteile sind im wesentlichen
darin zu sehen, daß der mechanische Aufwand für die hydraulische
Standphasensteuerung auf ein einziges bewegliches Bauelement reduziert wird, das mit einer Einrichtung zur Pendelphasendämpfung
in einfacher Weise kombiniert werden kann und die Gestaltung eines Hydraulikzylinders als einfachen Schubkolbentrieb mit nur
einem Kolben und einem Arbeitszylinder ermöglicht. Bauhöhe und
Masse des Hydraulikzylinders können mit Hilfe des beschriebenen
Prinzips der hydraulischen Stand- und Pendelphasensteuerung auf
ein geringstmögliches Maß reduziert werden, so daß auch unterhalb des Kniegelenks ein Winkeljustieradapter zum Abstimmen der
Prothesenstatik untergebracht werden kann und ein wichtiges Ziel
heutiger Prothesenversorgungen, insbesondere altersamputierten
Patienten mit Hilfe einer möglichst leichten und in der Standphase sicheren Prothese die Gehfähigkeit wiederzugeben, mit
dieser technischen Lösung leichter erreichbar wird.
Ein weiterer Vorteil der Erfindung besteht darin, daß trotz geringer Masse und Bauhöhe des Hydraulikzylinders unabhängig vom
Kniegelenk beliebige Fußtypen bewährter Bauart eingesetzt werden
können. Die Störanfälligkeit des Hydraulikzylinders kann durch
seine Gestaltung als geschlossenes System mit Hilfe eines
elastischen Balges gesenkt werden. Außerdem ermöglicht der
elastische Balg die Einsparung einer mechanischen Vorbringerfeder.

0056602

Patentansprüche:

1. Künstliches Kniegelenk mit hydraulischer Pendel- und Standphasensteuerung für Oberschenkelamputationen sowie Knie- und Hüftgelenkexartikulationen, bestehend aus einem Gelenkoberteil (1) mit einem Schaftansatz (2), einer Knieachse (4) und einem hinter dieser angelenkten oberen Achsbolzen (5), einem gabelförmigen Gelenkunterteil (9) mit unterem Achsbolzen (13), einem Justieradapter (15) und einem am oberen und unteren Achsbolzen (5, 13) hinter der Knieachse angelenkten Hydraulikzylinder (7), gekennzeichnet durch eine zylindrische Pendelhülse (21) mit kreisringförmiger unterer Stirnfläche und einer zentrisch verlaufenden und im oberen Ende endenden Bohrung (37), einer oberhalb der Bohrung befindlichen Abdichtung (39), einer Verstellvorrichtung zum axialen Verschieben der Pendelhülse, beispielsweise durch eine axial darüber in einem Innengewinde angeordnete Stellschraube oder eine Querbohrung (40), in deren untere Bohrungsaußenkante die Kegelansatzspitze eines Gewindestiftes (32) eingreift, durch eine elastische Scheibe (24), die unter der ringförmigen Stirnfläche der Pendelhülse oder einem Absatz der Pendelhülse im oberen Ende angeordnet die Pendelhülse nach oben drückt, durch ein Pendel (22) mit zylindrischen Querschnitt, das in der Bohrung (37) der Pendelhülse (21) am oberen Ende mittels einer zylindrischen Achse (42) drehbar gelagert ist, wobei die Enden der zylindrischen Achse mit der Pendelhülse fest verbunden sind und die Achse (42) waagerecht und quer zur Gehebene angeordnet ist, durch eine sich nach unten verbreiternde keilförmige Abrundung an der Vorderseite des Pendels (22a), eine am unteren Ende der Rückseite des Pendels senkrecht verlaufende ebene Dichtfläche (22b) und eine entgegen der Gehrichtung ansteigende untere Stirnfläche (22c) des Pendels, durch ein kurzes Röhrchen (43) mit ringförmiger Stirnfläche, das waagerecht in der Pendelhülse (21) so angeordnet ist, daß die ebene Dichtfläche (22b) des Pendels (22) die ringförmige Stirnfläche des Röhrchens (43) verschließen kann, durch eine etwa um einen halben Bohrungsdurchmesser zum kurzen Röhrchen (43) oder zur kreisringförmigen Stirnfläche der Pendelhülse (21) versetzten Zu- oder Austrittsbohrung, die beispielsweise in einer Kolbenstange (18, 44) oder einem Gehäuseteil des Hydraulikzylinders angeordnet durch Verkleinerung des freien Strömungsquerschnitts eine spaltblendenähnliche Drosselwirkung besitzt, durch Anordnung des Flüssigkeitseintritts von der Beugedruckseite des Hydraulikzylinders zur Pendelhülse (21) in der Weise, daß dieser stets von unten in die Bohrung (37) der Pendelhülse erfolgt und bei Anliegen der ebenen Dichtfläche (22b) des Pendels am Röhrchen (43) ein Schließdruck auf das Pendel wirkt, durch Ausrichtung von Pendel (22) und Pendelhülse (21) zum künstlichen Bein in der Weise, daß in Standposition oder leichter Schrittrücklageposition die ebene Dichtfläche (22b) des Pendels gerade vom Röhrchen (43) abhebt, durch einen Federstahldrahtstift (47), der an der oberen Vorder – seite des Pendels in einer rechtwinkligen Kerbe (46) mit dem Pendel fest verbunden ist, schräg nach vorne-unten in Gehrichtung zeigt und so lang ist, daß er beim Neigen des künstlichen Beins in der Schrittrücklage den Drehwinkel des Pendels begrenzt, durch einen Hydraulikzylinder mit nur einem Kolben und einem Arbeitszylinder, der entweder nach dem Schaltungsprinzip der einfachwirkenden Kolbenpumpe arbeitet und das Pendel mit Pendelhülse zentral in der Kolbenstange (18) aufnimmt oder der nach dem Prinzip der doppeltwirkenden Kolbenpumpe arbeitet und das Pendel mit Pendelhülse beispielsweise in einem Überström- und Kompensationsraum aufnimmt, der durch Umschließen des Arbeitszylinders von einem Außenzylinder, beispielsweise im Form eines elastischen Balges, entsteht.

2. Künstliches Kniegelenk mit hydraulischer Pendel- und Standphasensteuerung für Oberschenkelamputationen sowie Knie- und Hüftgelenkexartikulationen, bestehend aus einem Gelenkoberteil (1) mit einem Schaftansatz (2), einer Knieachse (4) und einem hinter dieser angelenkten oberen Achsbolzen (5), einem gabelförmigen Gelenkunterteil (9) mit unterem Achsbolzen (13), einem Justieradapter (15) und einem am oberen und unteren Achsbolzen (5, 13) hinter der Knieachse angelenkten Hydraulikzylinder (7), dadurch gekennzeichnet, daß in einem Hydraulikzylinder eine Kugel auf einer in der Gehebene liegenden und in Stand- oder leichter Schrittrücklageposition des künstlichen Beins zu diesem waagerecht angeordneten geraden Bahn frei beweglich gelagert ist, daß die gerade Bahn der Kugel hinten (in der Gehebene) durch eine Austrittsbohrung begrenzt wird, die auf Höhe des Kugelmittelpunktes der geraden Bahn angeordnet ist, daß der Weg des Hydrauliköls von der Beugedruckseite des Hydraulikzylinders vom Raum der Kugel aus durch diese Austrittsbohrung führt, so daß die Kugel infolge der Beinneigung in der Schrittvorlage vor die Austrittsbohrung rollt, diese verschließt und somit die Beugebewegung des Kniegelenks blockiert und infolge der entgegengesetzten Beinneigung in der Schrittrücklage wieder freigibt, daß eine Drosselschraube in Achsrichtung der Austrittsbohrung angebracht den freien Austrittsquerschnitt verändert, daß ein Hydraulikzylinder mit nur einem Kolben und einem Arbeitszylinder verwendet wird, der entweder nach dem Schaltungsprinzip der einfachwirkenden Kolbenpumpe arbeitet und die Kugel zentral im Kolben aufnimmt oder der nach dem Prinzip der doppeltwirkenden Kolbenpumpe arbeitet und die Kugel beispielsweise in einem unteren Gehäuseteil aufnimmt.

3. Künstliches Kniegelenk nach Anspruch 1, gekennzeichnet durch eine in Achsrichtung durchbohrte Kolbenstange (18) mit einer Bohrungsabstufung auf Höhe eines Kolbens (19) von einer großen Bohrung (34) auf eine kleine Bohrung (36), die nach unten durchgeht, wobei in der großen Bohrung (34) die Pendelhülse (21) und in der Abstufung der beiden Bohrungen (34, 36) die elastische Scheibe (24) mit zentraler Durchgangsbohrung und auf dieser von oben aufliegend ein rundes Rückschlagventilplättchen (23) oder eine Kugel aufgenommen wird, durch ein rundes Rückschlagventilplättchen (23), das auf der elastischen Scheibe (24) aufliegend sich im Bohrungsende (37) der Pendelhülse (21) befindet und einen geringeren Durchmesser als der Bohrung (37) der Pendelhülse, aber einen größeren Durchmesser, als die Durchgangsbohrung der elastischen Scheibe (24) aufweist, so daß zwischen Rückschlagventilplättchen und Bohrung der Pendelhülse ein kreisringförmiger Spielraum besteht, durch eine quer zur Achse der Kolbenstange (18) in Gehebene und entgegen der Gehrichtung unmittelbar über dem Kolben (19) angeordnete Durchgangsbohrung (44) in der Kolbenstange.

4. Künstliches Kniegelenk nach Anspruch 1 und 3, gekennzeichnet durch einen Arbeitszylinder (2o) mit einem am oberen Ende befindlichen spaltförmigen Schlitz (48), der sich nach außen keilförmig erweitert und über Bohrungen (5o) im oberen Deckel (27) Durchgang zu einem Kompensationsraum (51) besitzt, durch eine im oberen Deckel (27) befindliche Führungs- und Dichtungsbuchse (28) ohne elastische Dichtungselemente für die Kolbenstange (18), durch einen das untere Ende des Arbeitszylinders verschließenden unteren Deckel (25) mit einer elastischen Rückschlagklappe (26), die vom Arbeitszylinder aus eine senkrechte Bohrung (52) im Deckel (25) verschließt, die über zwei waagerechte Bohrungen (53) und senkrechte Kanäle (54) im Deckel (25) zum Kompensationsraum (51) Durchgang hat.

5. Künstliches Kniegelenk nach Anspruch 1, 3 und 4, gekennzeichnet durch einen elastischen Balg (29), der den gesamten Hydraulikzylinder umschließt, einen kreisförmigen Querschnitt hat, im Bereich des Arbeitszylinders (2o) zylindrisch, im mittleren Bereich der Kolbenstange (18) leicht kegelstumpfförmig und im oberen Bereich zylindrisch geformt ist, zur Abdichtung an der Kolbenstange (18) am oberen Ende auf den Außendurchmesser der Kolbenstange reduziert ist und hier mittels einer Schelle (31) gegen diese gepreßt wird und zur Abdichtung im unteren Bereich mit einer zweiten Schelle (3o) gegen den zylindrisch geformten Außenumfang des unteren Deckels (25) gepreßt wird, durch einen Kompensationsraum (51) zwischen dem elastischen Balg (29) und dem Arbeitszylinder (2o) bzw. der Kolbenstange (18), der so hoch mit Hydraulikflüssigkeit angefüllt ist, daß ein im oberen Bereich befindliches Luftvolumen durch den Arbeitshub des Hydraulikzylinders beim Durchschwingen des Prothesenunterschenkels stark komprimiert wird.

1/2

*Fig. 1*

0056602

2/2

Gehrichtung

Fig.3

Schnitt A-B

Fig.4

Fig.2

| Europäisches Patentamt | **EUROPÄISCHER RECHERCHENBERICHT** | Nummer der Anmeldung<br>EP 82 10 0155 |
|---|---|---|

| | **EINSCHLÄGIGE DOKUMENTE** | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | OELHYDRAULIK UND PNEUMATIK, Band 8, Nr.9, September 1964<br>J. ZICK: "Die Verwendung von hydraulischen Elementen in Prothesen"<br>Seiten 352-357<br><br>-- | 1 | A 61 F 1/04 |
| A | US - A - 2 480 856 (HENSCHKE)<br>* Spalte 9, Seiten 12-57; Figuren *<br><br>-- | 1 | |
| A | GB - A - 1 107 264 (ALLEN) | 1. | **RECHERCHIERTE SACHGEBIETE (Int. Cl.³)**<br><br>A 61 F |
| A | US - A - 4 212 087 (MORTENSEN)<br><br>---- | 1 | |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| X | Der vorliegende Recherchenbericht wurde für alle Patentansprüche erstellt. | | |
|---|---|---|---|
| Recherchenort<br>Den Haag | Abschlußdatum der Recherche<br>15-04-1982 | Prüfer<br>STEENBAKKER | |